# EUROPEAN PATENT APPLICATION

(11) **EP 1 306 052 A1**
(43) Date of publication of application: **02.05.2003**
(21) Application number: 02078529.1
(22) Date of filing: 27.08.2002
(51) Int. Cl.: A61B 5/145, A61B 5/15

(54) **Safety shielded needle assembly**

(30) Priority: 27.09.2001 US 965411
(71) Applicant: Becton, Dickinson and Company, Franklin Lakes, New Jersey 07417-1880 (US)
(72) Inventor: Newby, Marc, Tuxedo New York 10987 (US); Barkell, Paul, Plympton, Devon (GB)
(74) Representative: Iemenschot, Johannes Andreas, Ir.

(57) **Abstract**

The present invention is a safety shield assembly (10) having a shield (18) and a collar for connecting the shield (18) to a fluid handling device whereby the shield (18) may be pivoted with respect to the collar. The safety shield assembly (10) of the present invention also includes a thumb ramp (36) for orienting a user's hand with the safety shield assembly (10). Preferably, the safety shield assembly (10) may be used with a needle assembly, an intravenous infusion set, a syringe, a catheter or other fluid handling devices or assemblies that contain piercing elements.

## Description

The present invention relates to a shield which may be easily manipulated from an open to a closed position with respect to a needle, and more particularly to a safety shield assembly that may be used in conjunction with a syringe assembly, a hypodermic needle, a needle assembly, a needle assembly with a needle holder, a blood collection needle, a blood collection set, an intravenous infusion set or other fluid handling devices or assemblies that contain piercing elements.

Disposable medical devices having piercing elements for administering a medication or withdrawing a fluid, such as hypodermic needles, blood collecting needles, fluid handling needles and assemblies thereof, require safe and convenient handling. The piercing elements include, for example, pointed needle cannula or blunt ended cannula.

Safe and convenient handling of disposable medical devices is recognized by those in the medical arts as being necessary as to minimize exposure to blood borne pathogens. One manner in which to achieve safe and convenient handling of disposable medical devices is to dispose of the medical devices intact.

As a result of this recognition, numerous devices have been developed for shielding needles after use. Many of these devices are somewhat complex and costly. In addition, many of these devices are cumbersome to use in performing procedures. Furthermore, some of the devices are so specific that they preclude use of the device in certain procedures or with certain devices and/or assemblies.

Therefore, there exists a need for a syringe with a safety shield assembly: (i) that is easily manufactured; (ii) that is applicable to many devices; (iii) that is safe and simple to use with one hand; (iv) that can be safely disposed of; (v) that does not interfere with normal practices of needle use; (vi) that has tactile features whereby the user may be deterred from contacting the needle, the user may easily orient the needle with the patient and easily actuate and engage the shield assembly; (vii) that has visual features whereby the user may be deterred from contacting the needle, the user may easily orient the needle with the patient and easily actuate and engage the shield assembly; (viii) that is not bulky; (ix) that includes means for minimizing exposure to the user of residual fluid leaking from the needle; and (x) provides minimal exposure to the user because the needle shield is immediately initiated by the user after the needle is withdrawn from the patient's vein; and (xi) that is easy to activate without pushing the safety shield assembly against a hard surface, and (xi) which may be safely and easily manipulated between an open and closed position.

The present invention provides a safety shielded needle assembly that includes a needle holder, a needle extending from said needle holder, a shield pivotally connected to said needle holder for pivotal movement toward said needle, a finger pad for urging said shield toward said needle, and a thumb ramp adjacent to said finger pad to assure proper placement of hand with respect to said needle.

More specifically, the present invention provides a needle shield assembly and needle assembly. The needle assembly includes a hub and a needle connected to said hub. The needle includes a non-patient end and intravenous end having a bevel end. The needle shield assembly is connected to the needle assembly and includes a collar, a shield and a thumb ramp. The collar is connected to said hub of said needle assembly. The shield is movably connected to said collar whereby said shield by be pivoted with respect to said collar and easily moved between several positions with respect to said needle. The thumb ramp is connected to said hub adjacent to said shield to allow proper orientation of a user's hand.

Most preferably, the present invention provides a safety shield assembly including a safety shield. The safety shield uses tactile and visual means that includes touch pump end coloring for guiding the user's fingers to move the shield into various positions. A top finger guide area includes a first ramp that extends slightly on an upwardly slope from said rearward end of said shield to a shoulder. A thumb ramp is positioned adjacent to said shield to provide for proper orientation of a users hand.

Figure 1 shows a safety shielded needle assembly of the present invention.

Figures 2-6 show a hub for use with a safety shielded needle assembly of the present invention in front, top, side, bottom and end views.

Figures 7-11 show a safety shield for use with a safety shielded needle assembly of the present invention in front, top, side, bottom and end views.

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope and spirit of the invention. The scope of the invention will be measured by the appended claims and their equivalents.

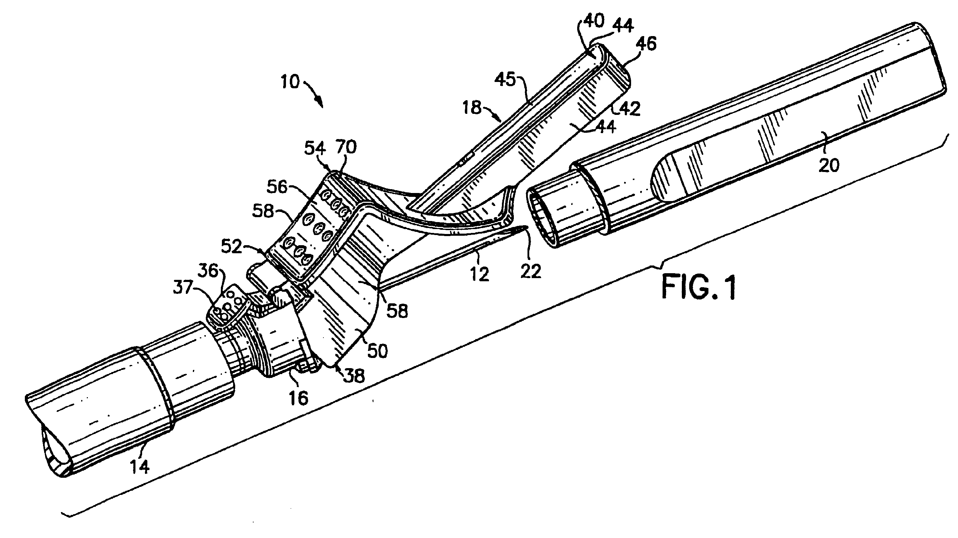

Referring to the drawings in which like reference numerals refer to like parts throughout, Figure 1 illustrates a safety shielded needle assembly 10 of the present invention. The shielded needle assembly 10 includes a cannular/needle 12, a syringe body 14 which supports the needle, a hub 16, a safety shield 18 pivotally connected to the hub, and a needle sleeve 20 which removably covers the patient end 22 of needle 12 prior to use. The needle 12 may be connected to syringe body in convention fashion such that the patient end 22 of needle 12 extends outwardly therefrom while a syringe body is shown. The present invention may be used in combination with any needle holder or similar device.

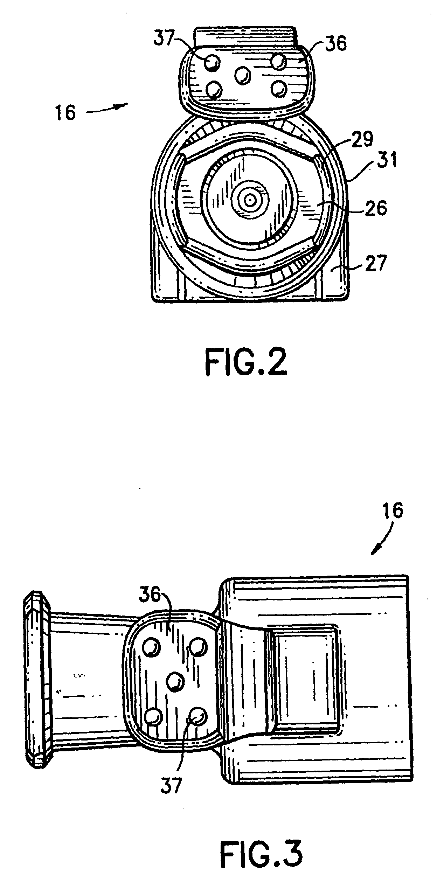

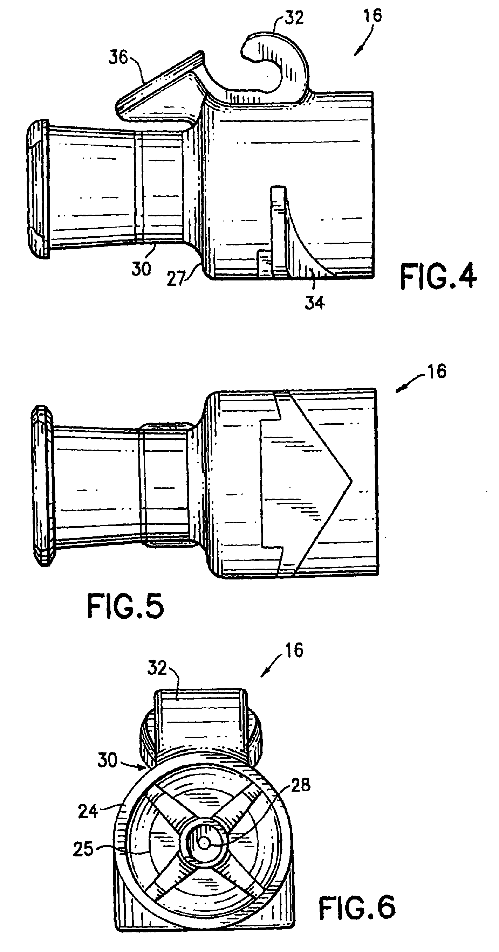

Referring additionally to Figures 2 through 6, hub 16 includes a forward annular skirt 24 and a rearward annular skirt 26. The forward annular skirt 24 is cylindrical and defines an inner side wall 25 and an outer side wall 30 and mates with rearward annular skirt 26 at shoulder 27. Rearward annular skirt 26 is cylindrical and includes an inner side wall 29 and an outer side wall 31 and extends from shoulder 27 opposite of forward annular skirt 24. The rearward annular skirt 26 may include a luer type connection for assembly onto a luer lock syringe. The forward annular skirt 24 includes a needle mount 28 for mounting needle 12 thereto in conventional fashion.

Extending from an outer sidewall 30 of forward skirt 24 is a hook member 32. Located opposite or downwardly of hook member 32 on outer sidewall 30 or opposed locking dents or protrusions 34. Extending rearwardly from hook member 32 on sidewall 30 is a thumb ramp 36 which will be described in detail hereinbelow.

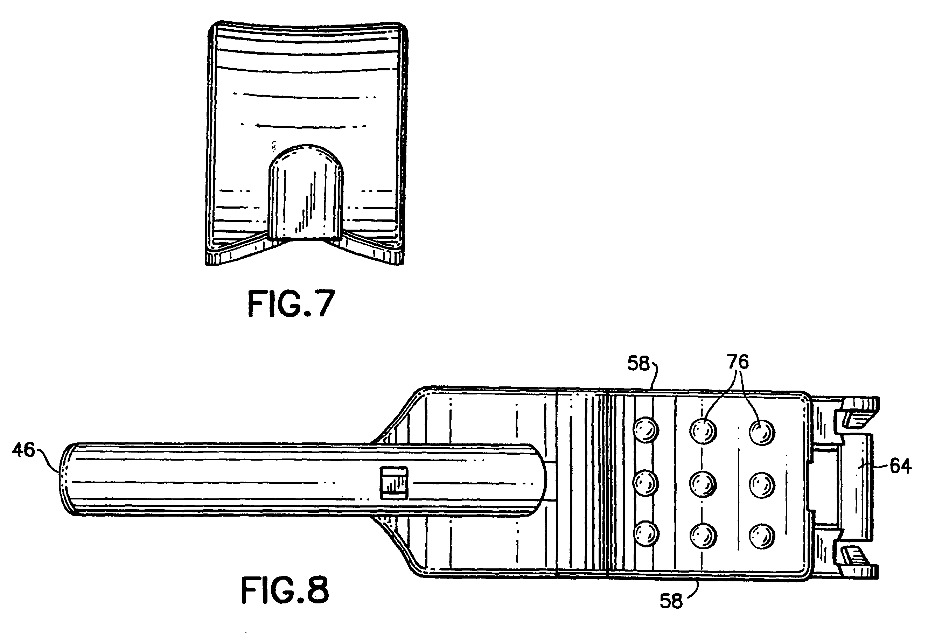

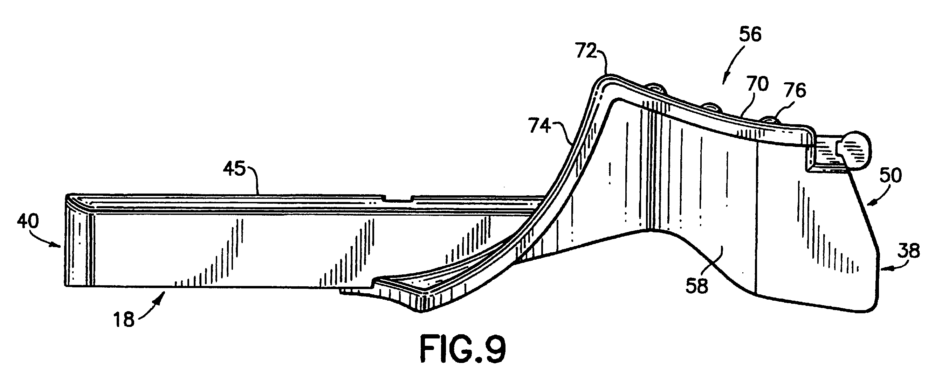

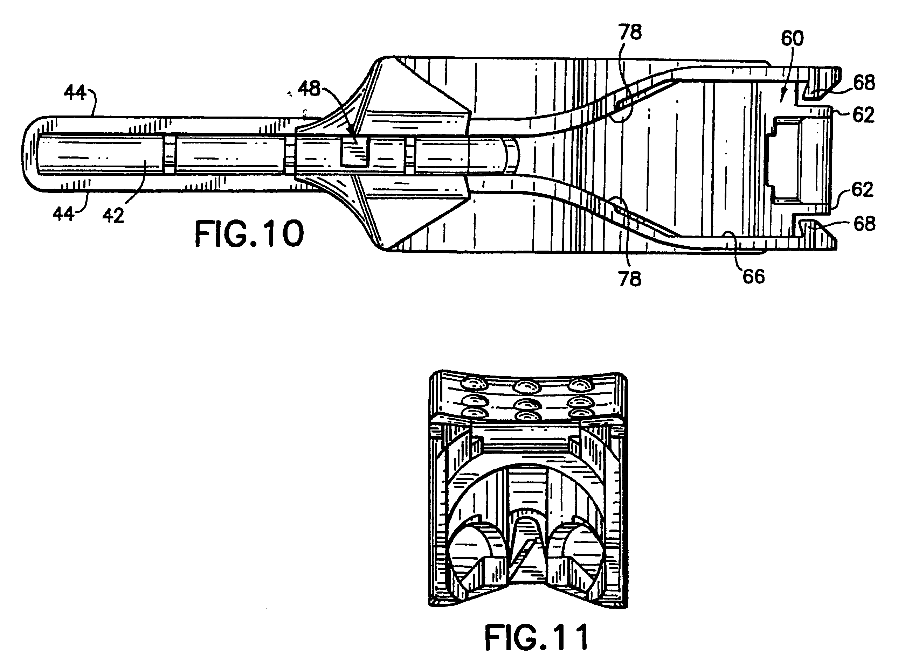

Referring additionally to Figures 7 through 11, safety shield 18 includes a rearward end 38 and a forward end 40.

Forward end 40 of shield 18 includes a slot or longitudinal opening 42 formed by sidewalls 44 that extend downwardly from a top section 45 and run substantially opposite of one another in parallel along the length of slot 42 toward forward end wall 46. The needle 12 may be trapped in slot 42 by the inclusion of an arm 48 that is located at one of the sidewalls 44 across slot 42 to secure the used needle 12.

Arm 48 is deflectable by the needle 12 when the needle enters the slot. Once the needle passes the end of the arm, the arm moves back to its original position whereby the needle is trapped within slot 42 by arm 48.

At the rearward end 38 of shield 18 is a hub engaging area 50 which is a continuation of slot 42. Hub engaging area 50 includes a rearward end 52 and a forward end 54, a top finger guide area 56, parallel sidewalls 58 that extend downwardly and inwardly from top finger guide area 56 and into sidewalls 44, an underside area 60 for surrounding hub 16 and extending arms 62 to hold hanger bar 64. Parallel sidewalls 58 include an inner surface 66 where barb dents 68 are located.

Top finger guide area 56 includes a first ramp 70 that extends slightly on an upward slope from the rear end of the hub engaging area 50 to a shoulder 72. Preferably, first ramp 70 includes one or more touch bumps 76 thereon. Touch bumps 76 provides a tactile and visual guide to alert the user that the user's finger has contacted the shield, and that the shield is in a defined or controlled position. Touch bumps 76 may be any configuration as long as they extend and are distinct from the top finger guide area. Touch bumps 76 may also be of distinguishing color as compared with the top finger guide area or shield.

Shield 18 further includes a second ramp 74 having an interior surface 78 for urging the needle towards the center of slot 42 as the shield is being rotated into the closed position. The exterior surfaces 58 are slightly inclined, extending radially from the second ramp. The interior surfaces 78 are especially helpful if the longitudinal axis of the needle is misaligned with respect to the longitudinal axis of the hub.

Extending arms 62 are located at rearward end 52 and at the beginning of top finger area 56 and hold hanger bar 64.

Located downwardly and extending from arms 62 and hanger bar 64, and on inner surface 66 of parallel sidewalls 58 are barb dents 68. Barb dents cooperate with locking dents on hub 16 to secure the shield in its final locked position.

In a preferred embodiment, the needle 12 is connected to syringe body 14 by use of hub 16. The needle 12 may be secured to the needle mount 28 of needle hub 16 which is then secured to syringe body 14 in conventional fashion. Joining techniques such as adhesive and ultrasonic welding may be employed. As assembled, the hook arm of the hub is aligned with the bevel up of the needle. The needle sleeve 20 is fitted over the needle 12 and is force-fitted over the forward skirt 24 of hub 16. Thereafter, shield 18 is connected to hub 16 whereby hanger bar 64 is force-fitted into hook member 32 whereby slot 42 faces needle sleeve 20. Preferably, the shield is connected to the hub by a force-fit or interference-fit between the hanger bar and the hook bar. Therefore, the shield is always oriented in a stable position. This allows the shield to be rotated and maintained in any position through its 270° arc of motion so that it does not interfere with any procedure for which the syringe is required. The shield will not move unless movement of the shield is positively initiated by the user. The shield is movable between two extreme positions of motion. These positions are fully opened when the shield is rotated to its maximum rearward position and fully closed whereby the shield is rotated fully forward and completely covers the needle. The shield can be maneuvered into any position inbetween and is held there by friction between hook member 32 and hanger bar 64. In the fully opened position, the needle can be clearly observed, allowing precise manipulation of the syringe and visibility of the needle bevel. In the fully closed position, the shield surrounds the point of the needle.

The safety shield assembly of the present invention further includes a thumb ramp 36 which is located on the hub 16 and is positioned adjacent to the first ramp 70 and spaced therefrom. As pressure is exerted on the first ramp 70, the shield 18 pivots relative to the hub 16 to cover the needle 12. The thumb ramp 36 provides a stable area of orientation for hand operating the safety shield 18, assuring that the user's hand not only will remain behind the needle but will reduce the likelihood of slipping during engagement of the shield. The thumb ramp 36 may also include a touch bump 37 similar to that on the first ramp 70. This will serve as an additional indicator that the user's hand is properly oriented with respect to the needle assembly 10.

When venipuncture is completed, the user easily rotates the shield from its open position towards the intravenous needle to an intermediate position. The user then pushes on the top finger guide area to move the shield to its final, unretractable locked position whereby the needle is trapped in the longitudinal opening within the arm of the needle shield and the barb dents of the shield, and held by locking dents of the hub 16. As the shield is pivoted, barb dents deflect over and are held by the locking dents.

## Claims

1. A safety shielded needle assembly comprising:
a needle holder;
a needle extending from said needle holder;
a shield pivotally connected to said needle holder for pivotal movement toward said needle;
a finger pad for urging said shield toward said needle; and
a thumb ramp adjacent to said finger pad to assure proper placement of hand with respect to said needle.

2. The assembly of claim 1, further including touch bumps on said thumb ramp for providing tactile indication of said proper hand placement.

3. The assembly of claim 1, wherein said thumb ramp is spaced for said finger pad.

4. A shielded needle assembly comprising:
a needle assembly including a hub and a needle connected to said hub said needle further including a non-patient end and an intravenous end having a beveled tip; and
a needle shield connected to said needle assembly having a collar, a shield and a thumb ramp, said collar being connected to said hub of said needle assembly, and said shield being movably connected to said collar, whereby said shield may be pivoted with respect to said collar and easily moved among several positions with respect to said needle, and said thumb ramp being connected to said hub adjacent to said shield to allow proper orientation of a user's hand.

5. The assembly of claim 4, further including tactile means on said shield and said thumb ramp for providing a user with a guide for pivoting said shield; said means comprising touch bumps.

6. The assembly of claim 4, where in said shield includes a rearward end, a forward end, a longitudinal slot for housing said needle in said forward end,
means for securing said needle in said slot;
means for guiding said needle into said slot, and
means for guiding the user's fingers to move said shield into various positions and means for retaining said shield over said needle.

7. The assembly of claim 6, wherein said means for guiding the user's fingers to move said shield into various positions includes a top finger guide area comprising a first ramp that extends slightly on an upwardly slope from said rearward end of said shield to a shoulder.

8. The assembly of claim 7, wherein said means for guiding said needle towards said slot includes a second ramp which slopes downwardly from said shoulder.

9. The assembly of claim 8, wherein said second ramp further includes an interior surface for urging said needle toward the center of the slot.

10. A safety shield assembly comprising:
a needle assembly comprising a hub, a needle connected to said hub comprising a non-patient end and an intravenous end comprising a bevel end;
a needle shield assembly movably connected to said needle assembly comprising a rearward end, a forward end, a collar and a shield whereby said collar is connected to said hub of said needle assembly and said shield is connected to said collar where by said shield may be pivoted with respect to said collar and easily moved in to several positions with respect to said needle; and
a needle holder having a distal end for receiving said non-patient end of said needle assembly;
wherein said safety shield assembly includes a top finger guide area including a first ramp that extends slightly on an upwardly slope from said rearward end of said shield to a shoulder, and a thumb ramp adjacent to said shield on said hub.

11. The assembly of claim 10, wherein said thumb ramp includes touch bumps.

12. The assembly of claim 10, wherein said shield further includes a slot or longitudinal opening for housing said intravenous end of said needle in said forward end, means for securing said intravenous end of said needle in said slot, means for guiding said intravenous end of said needle into said slot, means for connecting said shield and said collar, and means for retaining said shield over said intravenous end of said needle in a final non-retractable position.

13. The assembly of claim 10, wherein said first ramp includes at least one touch bump.

14. The assembly of claim 12, further including a means for guiding said intravenous end of said needle towards said slot.

15. The assembly of claim 14, wherein said means for guiding said intravenous end of said needle towards said slot includes a second ramp which slopes downwardly from said shoulder.

16. The assembly of claim 10, wherein said thumb ramp includes at least one touch bump.
